# EUROPEAN PATENT SPECIFICATION

(11) **EP 4 136 070 B1**
(45) Date of publication and mention of the grant of the patent: **29.05.2024**
(21) Application number: 21722548.1
(22) Date of filing: 06.04.2021
(51) Int. Cl.: C07C 259/10, C07C 271/60, C07C 281/06, C07C 337/02, C08F 301/00, C08F 26/00

(54) **MONOMERS, POLYMERS, AND ARTICLES FOR BIOMATERIAL CAPTURE**
MONOMERE, POLYMERE UND ARTIKEL ZUM EINFANGEN VON BIOMATERIAL
MONOMÈRES, POLYMÈRES ET ARTICLES POUR LA CAPTURE DE BIOMATÉRIAUX

(30) Priority: 14.04.2020 US 202063009547 P
(43) Date of publication of application: 22.02.2023
(73) Proprietor: Solventum Intellectual Properties Company, Maplewood, MN 55144 (US)
(72) Inventor: RASMUSSEN, Jerald K., Saint Paul, Minnesota 55133-3427 (US); NARVESON, Eli P., Louis Park, Minnesota 55426 (US)
(74) Representative: Mathys & Squire
(86) International application number: PCT/IB2021/052837
(87) International publication number: WO 2021/209859

(56) References cited:
- US-A1- 2017 183 299
- US-A1- 2018 217 035

## Description

### BACKGROUND

Detection, quantification, isolation, and purification of target biomaterials, such as viruses and biomacromolecules (including constituents or products of living cells, for example, proteins, carbohydrates, lipids, and nucleic acids) have long been objectives of investigators.

Detection and quantification are important diagnostically, for example, as indicators of various physiological conditions such as diseases. Isolation and purification of biomacromolecules are important for therapeutic uses and in biomedical research.

Polymeric materials have been widely used for the separation and purification of various target biomaterials. Such separation and purification methods can be based on any of a number of binding factors or mechanisms including the presence of an ionic group, the size of the target biomaterial, a hydrophobic interaction, an affinity interaction, the formation of a covalent bond, and so forth.

Membrane-based technologies, especially in disposable format, are becoming increasingly important in biopharmaceutical and vaccine manufacturing processes. Membranes have been used in passive, size-based separations (for example, in virus removal applications) and, more recently, in active filtration (for example, for the removal of minor contaminants in later stages of purification processes).

Ligand-functionalized substrates (particularly, ligand-functionalized membranes) having relatively high biomaterial binding capacities are particularly desirable. Ion exchange monomers derived from vinyldimethylazlactone (VDM) and isocyanatoethylmethacrylate (IEM) are known to provide grafted substrates with enhanced binding capacities for biological substances. When grafted as copolymers with conventional, commercially available comonomers, dramatic reduction in binding capacity is often observed. Thus, new monomers are desired that do not reduce binding capacities of such ion exchange monomers, or that reduce the binding capacity to a lesser extent.

### SUMMARY OF THE DISCLOSURE

The present disclosure provides monomers, polymers formed from such monomers, and articles for biomaterial capture including such polymers.

In one embodiment, the present disclosure provides a monomer represented by the following general Formula (I): CH₂=CR¹-C(=O)-X-R²-Z-X³-NR³-C(=X²)-X¹-R⁴, wherein: R¹ is H or -CH₃; R² is a (hetero)hydrocarbylene; X is -O- or -NH-; X¹ is -O-, -S-, -NH-, or a single bond; X² is -O- or -S-; X³ is -O- or -NR⁵-; R⁴ is hydrogen, (hetero)hydrocarbyl, or -N(R³)₂; each R³ and R⁵ is independently hydrogen or a (hetero)hydrocarbyl); and Z is -C(=O)- or -NH-C(=O)-.

In another embodiment, the present disclosure provides a polymer that includes interpolymerized units of at least one monomer of Formula (I).

Such polymer may be a copolymer and include interpolymerized units of at least one monomer represented by the following general Formula (XII):
CH₂=CR²¹-C(=O)-X²⁰-R²²-Z²⁰-X²³-Y²⁰, wherein: R²¹ is H or -CH₃; R²² is a (hetero)hydrocarbylene; X²⁰ is -O- or -NH-; X²³ is -O- or -NR²⁵-;
Z²⁰ is -C(=O)- or -NH-C(=O)-; Y²⁰ is hydrogen, (hetero)hydrocarbyl, or
-NR²³-C(=X²²)-X²¹-R²⁴; X²¹ is -O-, -S-, -NH-, or a single bond; X²² is -O- or -S-; R²⁴ is hydrogen, (hetero)hydrocarbyl, or -N(R²³)₂; and each R²³ and R²⁵ is independently hydrogen or a (hetero)hydrocarbyl. Such polymer may be grafted to a substrate.

In another embodiment, the present disclosure provides an article for biomaterial capture that includes a porous polymeric substrate; and borne on the porous substrate, a polymer as described herein.

As used herein, "alkyl" refers to a monovalent group that is a radical of an alkane and includes straight-chain, branched, cyclic, and bicyclic alkyl groups, and combinations thereof, including both unsubstituted and substituted alkyl groups. Unless otherwise indicated, the alkyl groups typically contain from 1 to 30 carbon atoms. In some embodiments, the alkyl groups contain 1 to 20 carbon atoms, 1 to 12 carbon atoms, 1 to 10 carbon atoms, 1 to 6 carbon atoms, 1 to 4 carbon atoms, or 1 to 3 carbon atoms. Examples of "alkyl" groups include, but are not limited to, methyl, ethyl, n-propyl, n-butyl, n-pentyl, isobutyl, t-butyl, isopropyl, n-octyl, n-heptyl, ethylhexyl, cyclopentyl, cyclohexyl, cycloheptyl, adamantyl, and norbornyl.

The term "alkylene" refers to a divalent group that is a radical of an alkane and includes groups that are linear, branched, cyclic, bicyclic, or a combination thereof. Unless otherwise indicated, the alkylene group typically has 1 to 30 carbon atoms. In some embodiments, the alkylene group has 1 to 20 carbon atoms, 1 to 12 carbon atoms, 1 to 10 carbon atoms, 1 to 6 carbon atoms, or 1 to 4 carbon atoms. In some embodiments, the alkylene is a linear saturated divalent hydrocarbon having from 1 to 12 carbon atoms, and in some embodiments, the alkylene is a branched saturated divalent hydrocarbon having from 3 to 12 carbon atoms, e.g., methylene, ethylene, propylene, 2-methylpropylene, pentylene, hexylene, 1,4-cyclohexylene, and 1,4-cyclohexyldimethylene.

The term "aryl" refers to a monovalent group that is aromatic and, optionally, carbocyclic. The aryl has at least one aromatic ring and may optionally include aralkyl or alkaryl groups. Any additional rings can be unsaturated, partially saturated, saturated, or aromatic. Optionally, the aromatic ring can have one or more additional carbocyclic rings that are fused to the aromatic ring. Unless otherwise indicated, the aryl groups typically contain from 6 to 30 carbon atoms. In some embodiments, the aryl groups contain 6 to 20, 6 to 18, 6 to 16, 6 to 12, or 6 to 10 carbon atoms. Examples of an aryl group include phenyl, benzyl, phenethyl, tolyl, chlorophenyl, methoxyphenyl, naphthyl, biphenyl, phenanthryl, and anthracyl.

The term "arylene" refers to a divalent group that is aromatic and, optionally, carbocyclic. The arylene has at least one aromatic ring. Optionally, the aromatic ring can have one or more additional carbocyclic rings that are fused to the aromatic ring. Any additional rings can be unsaturated, partially saturated, or saturated. In some embodiments, the arylene group has up to 5 rings, up to 4 rings, up to 3 rings, up to 2 rings, or one aromatic ring. For example, the arylene group can be phenylene. Unless otherwise specified, arylene groups often have 6 to 20 carbon atoms, 6 to 18 carbon atoms, 6 to 16 carbon atoms, 6 to 12 carbon atoms, or 6 to 10 carbon atoms.

The term "heteroatom" means an atom other than carbon or hydrogen.

The term "hydrocarbyl" is inclusive of aryl and alkyl. "Hydrocarbylene" is inclusive of arylene and alkylene.

The term "(hetero)hydrocarbyl" is inclusive of hydrocarbyl (alkyl and aryl) groups, and heterohydrocarbyl (heteroalkyl and heteroaryl) groups. Heterohydrocarbyl groups include one or more catenary (in-chain) heteroatoms, or one or more substituents comprising heteroatoms, such as oxygen, sulfur, or nitrogen atoms. Heterohydrocarbyl groups may optionally contain one or more catenary (in-chain) functional groups including ester, amide, urea, urethane, and carbonate functional groups. Unless otherwise indicated, the non-polymeric (hetero)hydrocarbyl groups typically contain from 1 to 60 carbon atoms, 1 to 40 carbon atoms, 1 to 20 carbon atoms, 1 to 10 carbon atoms, or 1 to 6 carbon atoms. Some examples of such heterohydrocarbyls as used herein include, but are not limited to, methoxyethyl, ethoxypropyl, propoxyethyl, 4-diphenylaminobutyl, 2-(2'-phenoxyethoxyl)ethyl, 3,6-dioxaheptyl, 3,6-dioxahexyl-6-phenyl, 2-imidazolyl, 3-furyl, and 3-indolmethyl

The term "(hetero)hydrocarbylene" is inclusive of hydrocarbylene (alkylene and arylene) groups, and heterohydrocarbylene (heteroalkylene and heteroarylene) groups Heterohydrocarbylene groups include one or more catenary (in-chain) heteroatoms or one or more substituents including heteroatoms, such as oxygen, sulfur, or nitrogen atoms. Heterohydrocarbylene groups may optionally contain one or more catenary (in-chain) functional groups including ester, amide, urea, urethane, and carbonate functional groups. Unless otherwise indicated, the non-polymeric (hetero)hydrocarbylene groups typically contain from 1 to 60 carbon atoms, 1 to 40 carbon atoms, 1 to 20 carbon atoms, 1 to 10 carbon atoms, or 1 to 6 carbon atoms. Some examples of such heterohydrocarbylenes as used herein include, but are not limited to, oxydiethylene, 3-thiabutylene, 3-diphenylaminobutylene, 2-(2'-phenoxyethoxyl)ethylene, 3,6-dioxaheptylene, 3,6-dioxahexyl-6-phenylene, 2,5-furylene, 2,6-pyridylene (also known as 2,6-pyridinediyl), and 2,5-thiophenedimethylene .

The term "hydrogen bond acceptor" means a heteroatom selected from oxygen, nitrogen, and sulfur that has a lone electron pair.

The term "hydrogen bond donor" means a moiety consisting of a hydrogen atom covalently bonded to a heteroatom selected from oxygen, nitrogen, and sulfur.

The term "ethylenically unsaturated group" refers to those groups having carbon-carbon double (or triple) bonds that may be free-radically polymerized, and includes (meth)acrylamides, (meth)acrylates, vinyl and vinyloxy groups, allyl and allyloxy groups, and acetylenic groups.

The term "carbonylimino" means a divalent group or moiety of formula -(CO)NR-, where R is hydrogen, alkyl (for example, selected from alkyl groups having from one to four carbon atoms), or aryl (preferably, hydrogen).

The term "iminocarbonylimino" means a divalent group or moiety of formula -N(R)-C(O)-N(R)-, wherein each R is independently hydrogen, alkyl (for example, selected from alkyl groups having from one to four carbon atoms), or aryl (preferably, at least one R is hydrogen; more preferably, both are hydrogen).

The term "iminothiocarbonylimino" means a divalent group or moiety of formula -N(R)-C(S)-N(R)-, wherein each R is independently hydrogen, alkyl (for example, selected from alkyl groups having from one to four carbon atoms), or aryl (preferably, at least one R is hydrogen; more preferably, both are hydrogen).

The term "oxycarbonylimino" means a divalent group or moiety of formula -O-C(O)-N(R)-, wherein R is hydrogen, alkyl (for example, selected from alkyl groups having from one to four carbon atoms), or aryl (preferably, hydrogen).

The term "oxythiocarbonylimino" means a divalent group or moiety of formula -O-C(S)-N(R)-, wherein R is hydrogen, alkyl (for example, selected from alkyl groups having from one to four carbon atoms), or aryl (preferably, hydrogen).

The term "thiocarbonylimino" means a divalent group or moiety of formula -(CS)NR-, where R is hydrogen, alkyl (for example, selected from alkyl groups having from one to four carbon atoms), or aryl (preferably, hydrogen).

The terms "polymer" and "polymeric material" include, but are not limited to, organic homopolymers, copolymers, such as for example, block, graft, random and alternating copolymers, terpolymers, etc., and blends and modifications thereof. Furthermore, unless otherwise specifically limited, the term "polymer" shall include all possible geometrical configurations of the material. These configurations include, but are not limited to, isotactic, syndiotactic, and atactic symmetries.

The term "comprises" and variations thereof do not have a limiting meaning where these terms appear in the description and claims. Such terms will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they materially affect the activity or action of the listed elements. Any of the elements or combinations of elements that are recited in this specification in open-ended language (e.g., comprise and derivatives thereof), are considered to additionally be recited in closed-ended language (e.g., consist and derivatives thereof) and in partially closed-ended language (e.g., consist essentially, and derivatives thereof).

The words "preferred" and "preferably" refer to embodiments of the disclosure that may afford certain benefits, under certain circumstances. However, other claims may also be preferred, under the same or other circumstances. Furthermore, the recitation of one or more preferred claims does not imply that other claims are not useful and is not intended to exclude other claims from the scope of the disclosure.

In this application, terms such as "a," "an," and "the" are not intended to refer to only a singular entity, but to include the general class of which a specific example may be used for illustration. The terms "a," "an," and "the" are used interchangeably with the term "at least one." The phrases "at least one of" and "comprises at least one of" followed by a list refers to any one of the items in the list and any combination of two or more items in the list.

As used herein, the term "or" is generally employed in its usual sense including "and/or" unless the content clearly dictates otherwise.

The term "and/or" means one or all of the listed elements or a combination of any two or more of the listed elements.

Also herein, all numbers are assumed to be modified by the term "about" and in certain embodiments, preferably, by the term "exactly." As used herein in connection with a measured quantity, the term "about" refers to that variation in the measured quantity as would be expected by the skilled artisan making the measurement and exercising a level of care commensurate with the objective of the measurement and the precision of the measuring equipment used. Herein, "up to" a number (e.g., up to 50) includes the number (e.g., 50).

Also herein, the recitations of numerical ranges by endpoints include all numbers subsumed within that range as well as the endpoints (e.g., 1 to 5 includes 1, 1.5, 2, 2.75, 3, 3.80, 4, 5, etc.).

As used herein, the term "room temperature" refers to a temperature of 20°C to 25°C or 22°C to 25°C.

The term "in the range" or "within a range" (and similar statements) includes the endpoints of the stated range.

Groupings of alternative elements or embodiments disclosed herein are not to be construed as limitations. Each group member may be referred to and claimed individually or in any combination with other members of the group or other elements found therein. It is anticipated that one or more members of a group may be included in, or deleted from, a group for reasons of convenience and/or patentability. When any such inclusion or deletion occurs, the specification is herein deemed to contain the group as modified thus fulfilling the written description of all Markush groups used in the appended claims.

When a group is present more than once in a formula described herein, each group is "independently" selected, whether specifically stated or not. For example, when more than one Y group is present in a formula, each Y group is independently selected. Furthermore, subgroups contained within these groups are also independently selected. For example, when each Y group contains an R, then each R is also independently selected.

Reference throughout this specification to "one embodiment," "an embodiment," "certain embodiments," or "some embodiments," etc., means that a particular feature, configuration, composition, or characteristic described in connection with the embodiment is included in at least one embodiment of the invention. Thus, the appearances of such phrases in various places throughout this specification are not necessarily referring to the same embodiment of the invention. Furthermore, the particular features, configurations, compositions, or characteristics may be combined in any suitable manner in one or more embodiments.

The above summary of the present disclosure is not intended to describe each disclosed embodiment or every implementation of the present invention. The description that follows more particularly exemplifies illustrative embodiments. In several places throughout the application, guidance is provided through lists of examples, which examples may be used in various combinations. In each instance, the recited list serves only as a representative group and should not be interpreted as an exclusive list. Thus, the scope of the present disclosure should not be limited to the specific illustrative structures described herein, but rather extends at least to the structures described by the language of the claims. Any of the elements that are positively recited in this specification as alternatives may be explicitly included in the claims or excluded from the claims, in any combination as desired. Although various theories and possible mechanisms may have been discussed herein, in no event should such discussions serve to limit the claimable subject matter.

### DETAILED DESCRIPTION OF ILLUSTRATIVE EMBODIMENTS

The present disclosure provides monomers, polymers formed from such monomers, and articles for biomaterial capture including such polymers.

The monomers of the present disclosure are hydrophilic and contain multiple hydrogen bond donors and acceptors. These monomers are useful for providing polymers with increased amounts of interchain hydrogen bonding interactions and intrachain hydrogen bonding interactions (i.e., increased H-bonding between adjacent monomers units).

These hydrophilic monomers are useful for surface modification of membranes, e.g., hydrophobic membranes, and nonwovens. They may be used as viral reduction membranes.

Also, because of their extensive capacities for hydrogen bonding, they are useful for the preparation of hydrogels, with possible application to wound dressings, etc.

They are useful in forming functionalized membranes and nonwovens that may be used in biopharmaceutical purifications. In particular, when used as comonomers with isocyanatoethylmethacrylate-based ion exchange monomers as described in U.S. Pat. No. 10,352,835 (Rasmussen et al.), they may be used to provide grafted copolymers having very high protein binding capacities. While not being bound by theory, it is believed that the reactivity ratios of the new monomers are more similar to that of the ionic monomers, thus resulting in more random or statistical copolymer formation.

### Monomers

The present disclosure provides a monomer (i.e., an uncharged monomer) represented by the following general Formula (I):

CH₂=CR¹-C(=O)-X-R²-Z-X³-NR³-C(=X²)-X¹-R⁴ (I)

wherein:
R¹ is H or -CH₃;
R² is a (hetero)hydrocarbylene;
X is -O- or -NH-;
X¹ is -O-, -S-, -NH-, or a single bond;
X² is -O- or -S-;
X³ is -O- or -NR⁵-;
R⁴ is hydrogen, (hetero)hydrocarbyl, or -N(R³)₂;
each R³ and R⁵ is independently hydrogen or a (hetero)hydrocarbyl; and
Z is -C(=O)- or -NH-C(=O)-.

In certain embodiments of Formula (I), R¹ is -CH₃.

In certain embodiments of Formula (I), R² is a (C1-C10)(hetero)hydrocarbylene, a (C1-C6)(hetero)hydrocarbylene, or a (C1-C4)(hetero)hydrocarbylene. In certain embodiments, R² is a (C1-C10)(hetero)alkylene, a (C1-C6)(hetero)alkylene, or a (C1-C4)(hetero)alkylene (i.e., an alkylene or a heteroatom-containing alkylene). In certain embodiments, R² is a (C1-C10)alkylene, a (C1-C6)alkylene, or a (C1-C4)alkylene.

In certain embodiments of Formula (I), X is -O-.

In certain embodiments of Formula (I), X¹ is -NH- or a single bond.

In certain embodiments of Formula (I), R⁴ is H when X¹ is -NH-. In certain embodiments of Formula (I), R⁴ is a (hetero)hydrocarbyl when X¹ is a single bond. In certain embodiments of Formula (I), R⁴ is a hydrocarbyl when X¹ is a single bond. In certain embodiments of Formula (I), R⁴ is methyl or phenyl when X¹ is a single bond.

In certain embodiments of Formula (I), X² is -O-.

In certain embodiments of Formula (I), X³ is -NR⁵-.

In certain embodiments of Formula (I), R⁵ is hydrogen.

In certain embodiments of Formula (I), R³ is hydrogen.

In certain embodiments of Formula (I), Z is -NH-C(=O)-.

In certain embodiments, the monomer of Formula (I) may be prepared from reaction of an alkenyl azlactone of general Formula (II) with a nucleophilic group-containing compound of general Formula (IV). In certain embodiments, the monomer of Formula (I) may be prepared from reaction of an ethylenically unsaturated isocyanate of general Formula (III) with a nucleophilic group-containing compound of general Formula (IV).

The alkenyl azlactone compound of Formula (II) is: 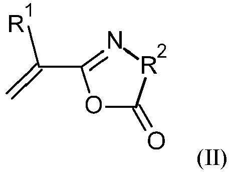 wherein:
R¹ is hydrogen or methyl; and
R² is (hetero)hydrocarbylene.

The ethylenically unsaturated isocyanate of general Formula (III) is:

CH₂=C(R¹)-C(=O)-X-R²-N=C=O (III)

wherein:
R¹ is hydrogen or methyl; and
R² is (hetero)hydrocarbylene.

The nucleophilic group-containing compound of general Formula (IV) is:

H-X³-N(R³)-C(=X²)-X¹-R⁴ (IV)

wherein:
X¹ is -O-, -S-, -NH-, or a single bond;
X² is -O- or -S-;
X³ is -O- or -NR⁵-;
R⁴ is hydrogen, (hetero)hydrocarbyl, or -N(R³)₂; and
each R³ and R⁵ is independently hydrogen or (hetero)hydrocarbyl.

In certain embodiments, the nucleophilic group-containing compound of Formula (IV) is selected from:
(thio)semicarbazides of general Formula (V) R⁴-NH-C(=X²)-N(R³)-N(R⁵)-H;
(thio)hydrazides of general Formula (VI) R⁴-C(=X²)-N(R³)-N(R⁵)-H;
(thio)hydroxamic acids of general Formula (VII) R⁴-C(=X²)-N(R³)-OH;
carbazates of general Formula (VIII) R⁴-O-C(=O)-N(R³)-N(R⁵)-H;
dithiocarbazates of general Formula (IX) R⁴-S-C(=S)-N(R³)-N(R⁵)-H; and
hydroxy(thio)ureas of general Formula (X) R⁴-NH-C(=X²)-N(R³)-OH.

In many instances, the monomers can be prepared by reaction in aqueous media in high yield and purity, such that the resultant solution can be utilized without further purification. In some instances, the monomers precipitate from the aqueous reaction mixture, and may be isolated by filtration. Alternatively, additional water may be added to redissolve the monomers. In some instances, the monomer can be prepared in nonaqueous organic solvents (e.g., ethyl acetate, dichloromethane, etc.) and isolated be removal of the organic solvent, such as by evaporation. Exemplary conditions for making such monomers are illustrated in the Examples Section.

In certain embodiments, the monomer of Formula (I) is (Monomer A):

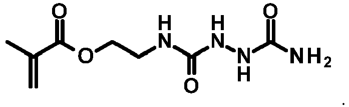

In certain embodiments, the monomer of Formula (I) is (Monomer B):

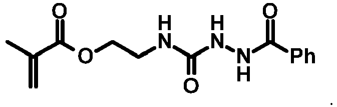

In certain embodiments, the monomer of Formula (I) is (Monomer C):

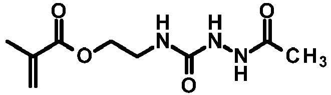

In certain embodiments, the monomer of Formula (I) is (Monomer E):

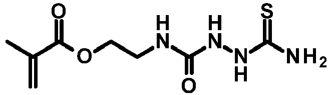

In certain embodiments, the monomer of Formula (I) is (Monomer F):

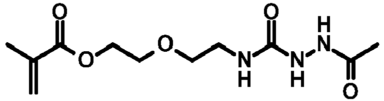

In certain embodiments, the monomer of Formula (I) is (Monomer G):

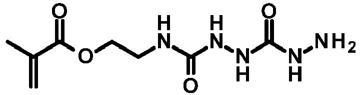

In certain embodiments, the monomer of Formula (I) is (Monomer H):

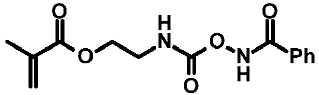

In certain embodiments, the monomer of Formula (I) is (Monomer I):

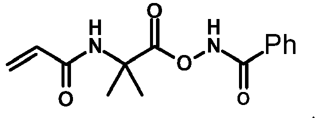

In certain embodiments, the monomer of Formula (I) is (Monomer J):

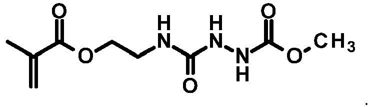

### Polymers

The above-described monomers generally can be homopolymerized. A skilled artisan will recognize, however, that the monomers can be copolymerized with other monomers (hereinafter, termed "comonomers"). Thus, the present disclosure provides a polymer (e.g., a homopolymer or copolymer) that includes interpolymerized units of at least one monomer of Formula (I). Such polymers may be a copolymer formed from two or more different monomers, selected, for example, to provide binding capacity, to adjust binding capacity, and/or to achieve special properties as desired.

In certain embodiments, the polymers described herein include 5 wt-% to 75 wt-% interpolymerized units of at least one monomer represented by Formula (I) (e.g., Monomers A-C and E-J).

In certain embodiments, the copolymers of the present disclosure may further include (e.g., 25 wt-% to 95 wt-%) interpolymerized units of at least one monomer (e.g., a charged monomer) of the following general Formula (XI):

CH₂=CR¹¹-C(=O)-X-R¹²-[Z¹⁻R¹²]ₙ-L (XI)

wherein:
R¹¹ is selected from hydrogen, alkyl, aryl, and combinations thereof;
each R¹² is independently a (hetero)hydrocarbylene;
X is -O- or -NR¹³-, where R¹³ is hydrogen or a (hetero)hydrocarbyl;
Z¹ is a (hetero)hydrocarbylene comprising at least one hydrogen bond donor, at least one hydrogen bond acceptor, or a combination thereof;
n is an integer of 0 or 1; and
L is a heteroatom-containing group comprising at least one monovalent ligand functional group selected from acidic groups, basic groups, and salts thereof.

In certain embodiments of Formula (XI), R¹¹ is hydrogen or alkyl. In certain embodiments of Formula (XI), R¹¹ is hydrogen or (C1-C4)alkyl. In certain embodiments of Formula (XI), R¹¹ is hydrogen or methyl.

In certain embodiments of Formula (XI), each R¹² is independently a hydrocarbylene. In certain embodiments of Formula (XI), each R¹² is independently an alkylene.

In certain embodiments of Formula (XI), X is -O- or -NR¹³-, where R¹³ is hydrogen.

In certain embodiments of Formula (XI), Z¹ is a (hetero)hydrocarbylene comprising at least one hydrogen bond donor and at least one hydrogen bond acceptor.

In certain embodiments of Formula (XI), Z¹ is a heterohydrocarbylene. In certain embodiments, such heterohydrocarbylene includes at least one moiety selected from carbonyl, carbonylimino, carbonyloxy, ether oxygen, thiocarbonylimino, iminocarbonylimino, iminothiocarbonylimino, oxycarbonylimino, oxythiocarbonylimino, and combinations thereof. In certain embodiments, such heterohydrocarbylene includes at least one moiety selected from carbonyl, carbonylimino, carbonyloxy, ether oxygen, iminocarbonylimino, oxycarbonylimino, and combinations thereof. In certain embodiments, such heterohydrocarbylene includes at least one moiety selected from carbonylimino, carbonyloxy, ether oxygen, iminocarbonylimino, and combinations thereof. In certain embodiments, such heterohydrocarbylene includes at least one moiety selected from carbonylimino, iminocarbonylimino, and combinations thereof.

In certain embodiments of Formula (XI), n is an integer of 1.

In certain embodiments of Formula (XI), L is a heteratom-containing acidic group or salt thereof. Exemplary L groups are selected from a carboxy group, a phosphono group, a phosphate group, a sulfono group, a sulfato group, a boronato group, combinations thereof, and salts thereof.

In certain embodiments of Formula (XI), L is a heteroatom-containing basic group or salt thereof. Exemplary L groups are selected from primary amino groups, secondary amino groups, tertiary amino groups, quaternary amino groups, guanidino groups, combinations thereof, and salts thereof.

Monomers of Formula (XI) are described, for example, in U.S. Pat. Nos. 10,353,835 (Rasmussen et al.), 10,239,828 (Rasmussen et al.), 9,616,394 (Bothof et al.), 9,272,246 (Rasmussen et al.), and 8,846,203 (Bothof et al.).

The present disclosure also provides a copolymer that includes 5 wt-% to 75 wt-% interpolymerized units of at least one monomer represented by the following general Formula (XII):

CH₂=CR²¹-C(=O)-X²⁰-R²²-Z²⁰-X²³-Y²⁰ (XII)

wherein:
R²¹ is H or -CH₃;
R²² is a (hetero)hydrocarbylene;
X²⁰ is -O- or -NH-;
X²³ is -O- or -NR²⁵-;
Z²⁰ is -C(=O)- or -NH-C(=O)-;
Y²⁰ is hydrogen, (hetero)hydrocarbyl, or -NR²³-C(=X²²)-X²¹-R²⁴;
X²¹ is -O-, -S-, -NH-, or a single bond;
X²² is -O- or -S-;
R²⁴ is hydrogen, (hetero)hydrocarbyl, or -N(R²³)₂; and
each R²³ and R²⁵ is independently hydrogen or a (hetero)hydrocarbyl.

In certain embodiments, the monomer of Formula (XII) is a monomer of Formula (I). In certain embodiments, the monomer of Formula (XII) is Monomer (D):

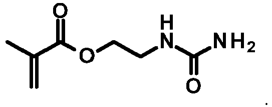

In certain embodiments, the copolymers of the present disclosure that include interpolymerized units of at least one monomer represented by the general Formula (XII), may further include (e.g., 25 wt-% to 95 wt-%) interpolymerized units of at least one monomer (e.g., a charged monomer) of the general Formula (XI).

Polymers of the present disclosure may be prepared using standard techniques, which are exemplified in the Examples Section.

Polymerization of the monomer(s) can be carried out by using known techniques. For example, the polymerization can be initiated with either a thermal initiator or a photoinitiator (preferably, a photoinitiator). Essentially any conventional free radical initiator can be used to generate the initial radical. Examples of suitable thermal initiators include peroxides such as benzoyl peroxide, dibenzoyl peroxide, dilauryl peroxide, cyclohexane peroxide, methyl ethyl ketone peroxide, hydroperoxides (for example, tert-butyl hydroperoxide and cumene hydroperoxide), dicyclohexyl peroxydicarbonate, t-butyl perbenzoate; 2,2,-azo-bis(isobutyronitrile); and combinations thereof. Examples of commercially available thermal initiators include initiators available from DuPont Specialty Chemical (Wilmington, DE) under the VAZO trade designation including VAZO 67 (2,2'-azo- bis(2-methylbutyronitrile)), VAZO 64 (2,2'-azo-bis(isobutyronitrile)), and VAZO 52 (2,2'- azo-bis(2,2-dimethylvaleronitrile)), as well as benzoylperoxide available under the trade designation LUCIDOL 70 from Elf Atochem North America, Philadelphia, PA.

Useful photoinitiators include benzoin ethers such as benzoin methyl ether and benzoin isopropyl ether; substituted acetophenones such as 2, 2-dimethoxyacetophenone available as IRGACURE 651 photoinitiator (Ciba Specialty Chemicals), 2,2 dimethoxy-2-phenyl-l-phenylethanone available as ESACURE KB-l photoinitiator (Sartomer Co.; West Chester, PA), 1-[4-(2-hydroxyethoxy)phenyl]-2-hydroxy-2-methyl-1-propan-1-one available as IRGACURE 2959 (Ciba Specialty Chemicals), and dimethoxyhydroxyacetophenone; substituted a-ketols such as 2-methyl-2-hydroxy propiophenone; aromatic sulfonyl chlorides such as 2-naphthalene-sulfonyl chloride; photoactive oximes such as 1-phenyl-l,2-propanedione-2-(0-ethoxy- carbonyl)oxime; and combinations thereof. Particularly preferred among these are the substituted acetophenones (especially 1-[4-(2-hy droxyethoxy)phenyl]-2-hydroxy-2-methyl- 1-propan-1-one, IRGACURE 2959, due to its water solubility). A particularly useful polymerizable photoinitiator is a 1:1 adduct of 2-vinyl-4,4-dimethylazlactone and IRGACURE 2959, which can be prepared essentially as described in Example 1 of U.S. Pat. No. 5,506,279 (Babu et al). Other useful photoinitiators include hydrogen-abstracting (Type II) photoinitiators such as benzophenone, 4-(3-sulfopropyloxy)benzophenone sodium salt, Michler's ketone, benzil, anthraquinone, 5,12-naphthacenequinone, aceanthracenequinone, benz(A)anthracene-7,12-dione, 1,4-chrysenequinone, 6,13-pentacenequinone, 5,7,12,14-pentacenetetrone, 9-fluorenone, anthrone, xanthone, thioxanthone, 2-(3-sulfopropyloxy)thioxanthen-9-one, acridone, dibenzosuberone, acetophenone, chromone, and combinations thereof.

The initiator can be used in an amount effective to initiate free radical polymerization of the monomer(s). Such amount will vary depending upon, for example, the type of initiator and polymerization conditions utilized. The initiator generally can be used in amounts ranging from 0.01 part by weight to 5 parts by weight, based upon 100 parts total monomer.

The polymerization solvent can be essentially any solvent that can substantially dissolve (or, in the case of emulsion or suspension polymerizations, disperse or suspend) the monomer(s) (and comonomer(s), if used). In many embodiments, the solvent can be water or a water/water-miscible organic solvent mixture. The ratio of water to organic solvent can vary widely, depending upon monomer solubility. With some monomers, the ratio typically can be greater than 1:1 (volume/volume) water to organic solvent (preferably, greater than 5:1; more preferably, greater than 7:1). With other monomers, a higher proportion of organic solvent (even up to 100 percent) can be preferred (with some alcohols especially).

Any such water-miscible organic solvent preferably has no groups that would retard polymerization. In some embodiments, the water-miscible solvents can be protic group-containing organic liquids such as the lower alcohols having 1 to 4 carbon atoms, lower glycols having 2 to 6 carbon atoms, and lower glycol ethers having 3 to 6 carbon atoms and 1 to 2 ether linkages. In some embodiments, higher glycols such as polyethylene glycol) can be used. Specific examples include methanol, ethanol, isopropanol, n-butanol, t-butyl alcohol, ethylene glycol, methoxyethanol, ethoxyethanol, propoxyethanol, butoxyethanol, methyl carbitol, ethyl carbitol, and combinations thereof.

In other embodiments, non-protic water-miscible organic solvents can be used. Such solvents include aliphatic esters (for example, methoxyethyl acetate, ethoxyethyl acetate, propoxyethyl acetate, butoxyethyl acetate, and triethyl phosphate), ketones (for example, acetone, methyl ethyl ketone, and methyl propyl ketone), and sulfoxides (for example, dimethyl sulfoxide).

The monomer concentration in the polymerization solvent can vary, depending upon a number of factors including, but not limited to, the nature of the monomer or monomers, the extent of polymerization desired, the reactivity of the monomer(s), and the solvent used. Typically, the monomer concentration can range from 0.1 weight percent (wt-%) to 60 wt-% (preferably, from 1 wt-% to 40 wt-%; more preferably, from 5 wt-% to 30 wt-%), based upon the total weight of monomer and solvent.

An aqueous monomer mixture optionally can be formulated with relatively higher levels of multifunctional (crosslinking) monomers or comonomers (for example, from 5 wt-% to 90 wt-%, based upon the total weight of monomer(s) and comonomer(s)) and polymerized as a suspension or dispersion in a nonpolar, immiscible organic solvent, optionally in the presence of added porogen(s), to produce crosslinked, porous particles comprising the instant monomer(s).

Such methods are well known and are described, for example, in U.S. Pat. Nos. 7,098,253 (Rasmussen et al), 7,674,835 (Rasmussen et al), 7,674,836 (Rasmussen et al), and 7,683,100 (Rasmussen et al.).

If desired, the polymerization can be carried out in the presence of a porous substrate, so as to form an article comprising a porous substrate bearing the resulting polymer. For example, an imbibing or coating solution comprising the monomer(s), any comonomer(s), initiator(s), and solvent(s) can be imbibed by or coated (or otherwise deposited) on a porous substrate as described, for example, in U.S. Pat. No. 10,352,835 (Rasmussen et al.).

### Substrates

The polymers described herein may be disposed on (e.g., grafted to) a substrate, preferably a porous substrate and used in filter elements.

The porous substrate can be in essentially any form such as particles, fibers, films, webs, membranes, sponges, or sheets. Suitable porous substrates can be organic, inorganic, or a combination thereof (preferably, organic; more preferably, polymeric). Suitable porous substrates include porous particles, porous membranes, porous nonwoven webs, porous woven webs, porous sponges, porous fibers, and combinations thereof.

In certain embodiments, porous substrates include porous nonfibrous membranes as well as porous nonwoven webs and other porous fibrous substrates.

In certain embodiments, the porous substrate is a porous nonwoven web. As used herein, the terms "nonwoven web" or "nonwoven substrate" are used interchangeably and refer to a fabric that has a structure of individual fibers or filaments which are randomly and/or unidirectionally interlaid in a mat-like fashion.

A fibrous nonwoven web can be made by carded, air laid, spunlaced, spunbonding or melt-blowing techniques or combinations thereof. Spunbonded fibers are typically small diameter fibers that are formed by extruding molten thermoplastic polymers as filaments from a plurality of fine, usually circular capillaries of a spinneret with the diameter of the extruded fibers being rapidly reduced. Meltblown fibers are typically formed by extruding the molten thermoplastic material through a plurality of fine, usually circular, die capillaries as molten threads or filaments into a high velocity, usually heated gas (e.g. air) stream which attenuates the filaments of molten thermoplastic material to reduce their diameter. Thereafter, the meltblown fibers are carried by the high velocity gas stream and are deposited on a collecting surface to from a web of randomly disbursed meltblown fibers. Any of the nonwoven webs may be made from a single type of fiber or two or more fibers that differ in the type of thermoplastic polymer and/or thickness.

Suitable nonwoven substrates may be spunlaid, hydroentangled, or meltblown. In certain embodiments, they may have a tensile strength of at least 4.0 newtons prior to grafting, a surface area of 15 to 50 m² per square meter of nonwoven substrate, a mean pore size of 1-40 microns according to ASTM F 316-03, and a solidity of less than 20%.

The porous substrate may be formed from any suitable thermoplastic polymeric material. Suitable polymeric materials include, but are not limited to, polyolefins, poly(isoprenes), poly(butadienes), fluorinated polymers, chlorinated polymers, polyamides, polyimides, polyethers, poly(ether sulfones), poly(sulfones), poly(vinyl acetates), copolymers of vinyl acetate, such as poly(ethylene)-co-poly(vinyl alcohol), poly(phosphazenes), poly(vinyl esters), poly(vinyl ethers), poly(vinyl alcohols), and polycarbonates).

Suitable polyolefins include, but are not limited to, poly(ethylene), poly(propylene), poly(l-butene), copolymers of ethylene and propylene, alpha olefin copolymers (such as copolymers of ethylene or propylene with 1-butene, 1-hexene, 1-octene, and 1-decene), poly(ethylene-co-1-butene) and poly(ethylene-co-1-butene-co-1-hexene).

Suitable fluorinated polymers include, but are not limited to, poly(vinyl fluoride), poly(vinylidene fluoride), copolymers of vinylidene fluoride (such as poly(vinylidene fluoride-co-hexafluoropropylene), and copolymers of chlorotrifluoroethylene (such as poly(ethylene-co-chlorotrifluoroethylene).

Suitable polyamides include, but are not limited to, poly(iminoadipoyliminohexamethylene), poly(iminoadipoyliminodecamethylene), and polycaprolactam. Suitable polyimides include, but are not limited to, poly(pyromellitimide). Suitable poly(ether sulfones) include, but are not limited to, poly(diphenylether sulfone) and poly(diphenylsulfone-co-diphenylene oxide sulfone).

Suitable copolymers of vinyl acetate include, but are not limited to, poly(ethylene-co-vinyl acetate) and such copolymers in which at least some of the acetate groups have been hydrolyzed to afford various poly(vinyl alcohols).

In some embodiments, the porous substrate is formed from a propylene homo- or copolymers, most preferably propylene homopolymers. Polypropylene polymers are often a material of choice for porous articles, such as nonwovens and microporous films, due to properties such as non-toxicity, inertness, low cost, and the ease with which it can be extruded, molded, and formed into articles.

In certain embodiments, the porous substrate is a nonfibrous porous membrane. Examples may be formed from any suitable thermoplastic polymeric material as described above for the fibrous substrate.

In some embodiments, the nonfibrous porous membrane is a microporous membrane such as a thermally-induced phase separation (TIPS) membrane. TIPS membranes are often prepared by forming a homogenous solution of a thermoplastic material and a second material above the melting point of the thermoplastic material. Upon cooling, the thermoplastic material crystallizes and phase separates from the second material. The crystallized thermoplastic material is often stretched. The second material is optionally removed either before or after stretching. Microporous membranes are further disclosed in U.S. Pat. Nos. 4,539,256 (Shipman), 4,726,989 (Mrozinski), 4,867,881 (Kinzer), 5,120,594 (Mrozinski), 5,260,360 (Mrozinski et al.), and 5,962,544 (Waller). Further, the microporous film can be prepared from ethylene-vinyl alcohol copolymers as described in U.S. Pat. No. 5,962,544 (Waller).

Some exemplary TIPS membranes include poly(vinylidene fluoride) (PVDF), polyolefins such as polyethylene homo- or copolymers or polypropylene homo- or copolymers, vinyl-containing polymers or copolymers such as ethylene-vinyl alcohol copolymers and butadiene-containing polymers or copolymers, and acrylate-containing polymers or copolymers. TIPS membranes comprising PVDF are further described in U.S. Pat. No. 7,338,692 (Smith et al.).

In another exemplary embodiment, the porous nonfibrous membrane is a microporous membrane such as a solvent-induced phase separation (SIPS) membrane. SIPS membranes are often prepared by forming a homogenous solution of a thermoplastic material and a second material (a solvent), the solution is cast into a film or hollow fiber form, then immersed in a nonsolvent bath. The nonsolvent causes the thermoplastic material to solidify, or phase separate, and also extracts out the solvent, leaving a porous polymeric membrane. Examples of SIPS membranes prepared from polyamides include a nylon microporous film or sheet, such as those described in U.S. Pat. Nos. 6,056,529 (Meyering et al.), 6,267,916 (Meyering et al.), 6,413,070 (Meyering et al.), 6,776,940 (Meyering et al.), 3,876,738 (Marinacchio et al.), 3,928,517 (Knight et al.), 4,707,265 (Knight et al.), and 5,458,782 (Hou et al.). Other examples include microporous membranes prepared from polysulfones and polyethersulfones, many of which are commercially available from 3M Company, St. Paul, MN, MicroPES and DuraPES.

Polymers of the present disclosure may be disposed on (e.g., grafted to) a porous substrate using standard techniques, which are exemplified in the Examples Section, and are described in U.S. Pat. Nos. 10,352,835 (Rasmussen et al.), 9,821,276 (Berrigan et al.), and 8,846,203 (Bothof et al.).

### Articles

The polymers of the present disclosure are particularly useful in an article for biomaterial capture. Such articles include a porous polymeric substrate as described herein, and borne on such porous substrate, a polymer of the present disclosure. In certain embodiments, the polymer is grafted to the porous substrate. Such substrates with the polymers described herein borne thereon may serve as a filter element.

The coated or grafted polymer can alter the original nature of the porous substrate. The resulting polymer-bearing porous substrates can retain many of the advantages of the original porous substrate (for example, mechanical and thermal stability, porosity, and so forth) but can also exhibit enhanced affinity for biomaterials such as viruses, proteins, and the like. Porous substrates bearing the polymers described herein can be particularly useful as filter media for the selective binding and removal of target biomaterials or biological species (including relatively neutral or charged biomaterials such as viruses and other microorganisms, acidic carbohydrates, proteins, nucleic acids, endotoxins, bacteria, cells, cellular debris, and the like) from biological samples.

Articles comprising the polymer-bearing porous substrates can further include conventional components such as housings, holders, adapters, and combinations thereof.

If desired, efficiency of binding and capture of biomaterials can be improved by using a plurality of stacked or layered, functionalized porous substrates (for example, functionalized porous membranes) as a filter element. Thus, a filter element can comprise one or more layers of functionalized porous substrate. The individual layers of the filter element can be the same or different. The layers can vary in porosity, degree of grafting, and so forth. The filter element can further comprise an upstream prefilter layer and/or a downstream support layer. The individual layers can be planar or pleated, as desired.

Examples of suitable prefilter and support layer materials include any suitable porous membranes of polyethersulfone, polypropylene, polyester, polyamide, resin-bonded or binder-free fibers (for example, glass fibers), and other synthetics (woven and nonwoven fleece structures); sintered materials such as polyolefins, metals, and ceramics; yarns; special filter papers (for example, mixtures of fibers, cellulose, polyolefins, and binders); polymer membranes; and combinations thereof.

Useful articles for biomaterial capture or filtration applications include a filter cartridge including one or more of the above-described filter elements, a filter assembly comprising one or more of the above-described filter elements and a filter housing. The articles can be used in carrying out a method of capture or removal of a target biomaterial or biological species comprising (a) providing at least one article comprising at least one above-described filter element; and (b) allowing a moving biological solution containing a target biomaterial to impinge upon the upstream surface of the filter element for a time sufficient to effect binding of the target biomaterial.

### EXAMPLES

These Examples are merely for illustrative purposes and are not meant to be overly limiting on the scope of the appended claims. Notwithstanding that the numerical ranges and parameters setting forth the broad scope of the present disclosure are approximations, the numerical values set forth in the specific examples are reported as precisely as possible. Any numerical value, however, inherently contains certain errors necessarily resulting from the standard deviation found in their respective testing measurements. At the very least, and not as an attempt to limit the application of the doctrine of equivalents to the scope of the claims, each numerical parameter should at least be construed in light of the number of reported significant digits and by applying ordinary rounding techniques.

Unless otherwise noted, all parts, percentages, ratios, etc. in the examples and the rest of the specification are by weight, and all reagents used in the examples were obtained, or are available, from general chemical suppliers such as, for example, Sigma-Aldrich Company, Saint Louis, Missouri, or may be synthesized by conventional methods.

### Materials

4,4-Dimethyl-2-vinyl-4H-oxazol-5-one (vinyldimethylazlactone (VDM, CAS# 29513-266) was obtained from SNPE Incorporated, Princeton, NJ.
2-Isocyanatoethylmethacrylate (IEM) was obtained from Showa Denko, Fukushima, Japan, as Karenz MOI.
2-Isocyanatoethylacrylate (IEA) was obtained from Showa Denko, Fukushima, Japan, as Karenz AOI.
2-(2-Isocyanatoethoxy)ethylmethacrylate (MOI-EG) was obtained from Showa Denko, Fukushima, Japan, as Karenz MOI-EG.
4-(3-Sulfopropyloxy)benzophenone, sodium salt (S-BP) photoinitiator was prepared as described in U.S. Pat. No. 10,039,856 (Rasmussen et al.).

All other employed chemicals were obtained from commercial sources as reagent grade and were used as received.

### Protein Solutions

Human IgG (polyclonal immunoglobulin G, 153 kDa, pI = 6.4-9.1, Sigma-Aldrich) was prepared at a concentration of about 4.5 milligrams per milliliter (mg/mL) in 50 millimolar (mM) HEPES (N-(2-hydroxyethyl)piperazine-N'-(4-butanesulfonic acid)) buffer, pH 7.0.

### Monomer Synthesis

The IEM adduct of 4-aminobutyric acid (IEM/GABA) was prepared by the procedure described in Prep. Ex. 14 of U.S. Pat. No. 10,352,835 (Rasmussen et al.).

### Coating and UV Grafting

Coating solutions were prepared by mixing monomer solutions as prepared with deionized water and S-BP photoinitiator (varying amounts of a 0.05 gram per milliliter (g/mL) solution in deionized water) to provide a mixture of the desired monomer concentration (molar). Weight % solids of the synthesized monomer solutions were measured and used to calculate dilution protocols for each coating experiment. Coating solutions were applied to nylon membranes and UV grafted as described in U.S. Pat. No. 10,352,835 (Rasmussen et al.).

### Ligand Density and Ligand Molar Ratio

Ligand density was determined based on mass gained by the membrane after grafting. Briefly, the number of millimoles of ligand monomer grafted to each membrane substrate was calculated by dividing the mass gain by the molecular weight of the grafting monomer. For copolymers, the assumption was made that reactivity ratios were similar, and that mass gain associated with each monomer occurred in the same ratio as charged. The resulting ligand density was then normalized by dividing by the original mass of the substrate and expressed as millimoles of ligand grafted per gram of substrate (millimoles per gram (mmol/g)). The measured bulk density of the membrane (0.415 g/mL) was used to convert ligand density to a volumetric basis, and protein molecular weight was used to convert capacity to a molar basis. After determination of the static protein binding capacity, molar ratio of ligands per protein molecule was expressed as the quotient of static binding capacity to ligand density. These values were used to compare copolymers to the homopolymer in the Tables 1-3 below.

### Static (Equilibrium) Protein Binding Capacity Method for Functionalized Membranes

Functionalized membranes prepared as described above were analyzed in triplicate for static binding capacity by procedures described in U.S. Pat. No. 10,352,835 (Rasmussen et al.).

### Example 1. Synthesis of the IEM adduct of semicarbazide hydrochloride (Monomer A)

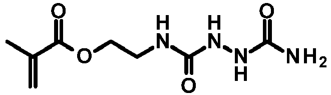

Semicarbazide hydrochloride (5.58 grams, 0.05 mol, obtained from Alfa Aesar, Ward Hill, MA) was charged to a 200 millilters (mL) round bottom flask, placed in an ice-water bath, and dissolved in 1 Normal (N) sodium hydroxide solution (50 mL) with magnetic stirring. The mixture was stirred for 20 minutes and then IEM was added (1.92 grams, 12.4 mmol). Three additional portions of IEM (1.92 grams each) were added after 10 minute intervals. The ice-water bath was removed and stirring was continued for one hour. The colorless precipitate was filtered and dried to give a quantitative yield of Monomer A. The product was soluble in trifluoracetic acid, as well as in acetic acid with warming. ¹H-NMR (trifluoracetic acid/CDCl₃): δ 1.97 (s, 3H), 3.75 (t, 2H), 4.46 (t, 2H), 5.78 (s, 1H), 6.25 (s, 1H), 7.00 (very broad s), 7.96 (broad s, 1H), 8.07 (broad s, 1H).

### Example 2. Synthesis of the IEM adduct of benzhydrazide (Monomer B)

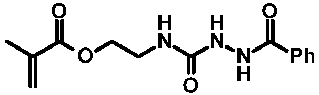

Benzhydrazide (6.81 grams, 0.05 mol, obtained from Alfa Aesar) was charged to a 200 mL round bottom flask and 100 mL of deionized water was added with magnetic stirring. Brief warming with a heat gun and continued stirring produced a clear, homogeneous solution. The flask was placed in an ice-water bath and the contents were stirred for two minutes. Next, IEM (1.92 grams) was added to the flask. Three additional portions of IEM (1.92 grams each) were subsequently added at 5 to 10 minute intervals. The reaction was stirred for ten minutes. The ice bath was removed and stirring was continued for one hour. The resulting precipitate was filtered and air dried to provide 13.4 grams (92%) of Monomer B ( soluble in methanol). ¹H-NMR (CDCl₃): δ 1.83 (s, 3H), 3.37 (m, 2H), 4.10 (t, 2H), 5.46 (m, 1H), 6.02 (s, 1H), 6.28 (t, 1H), 7.35 (m, 2H), 7.45 (m, 1H), 7.83 (m, 2H), 8.08 (broad s, 1H), 9.63 (broad s, 1H).

### Example 3. Synthesis of IEM adduct of acethydrazide (Monomer C)

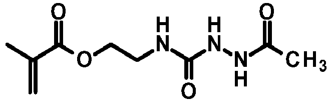

Acethydrazide (7.40 grams, 0.10 mol, Alfa Aesar) was charged to a 500 mL round bottom flask, placed in an ice-water bath, and dissolved in deionized water (200 mL) with magnetic stirring. The mixture was stirred for twenty minutes and then IEM was added (7.75 grams, 0.05 mol). The mixture was stirred for twenty minutes, followed by the addition of a second equivalent charge of IEM. The mixture was stirred overnight, filtered to remove a small amount of precipitate, and then diluted to 5% weight/weight (w/w) with deionized water. 1H-NMR (D2O): δ 1.74 (s, 3H), 1.85 (s, 3H), 3.31 (t, 2H), 4.05 (t, 2H), 5.54 (s, 1H), 5.95 (s, 1H).

### Example 4. Synthesis of IEM adduct of ammonia (Monomer D)

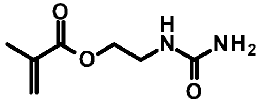

Concentrated ammonium hydroxide (6.76 mL, 0.10 mol ammonia, EMD Millipore, Burlington, MA) was charged to a 500 mL round bottom flask, placed in an ice-water bath, and diluted with deionized water (50 mL) with magnetic stirring. The mixture was stirred for ten minutes and then IEM was added (7.75 grams, 0.05 mol). The mixture was stirred for 20 minutes, followed by the addition of a second equivalent charge of IEM. The mixture was stirred overnight and filtered to remove a small amount of precipitate. 1H-NMR (D2O): δ 1.74 (s, 3H), 3.26 (t, 2H), 4.04 (t, 2H), 5.54 (s, 1H), 5.96 (s, 1H).

Moisture balance measurement indicated a 23% w/w solution.

### Example 5. Synthesis of IEM adduct of thiosemicarbazide (Monomer E)

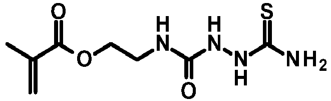

The general procedure described in Example 1 was followed with the exceptions that thiosemicarbazide was used in place of semicarbazide hydrochloride and deionized water was used in place of 1N sodium hydroxide solution. The colorless product crystallized from the reaction mixture and was isolated by filtration. ¹H-NMR (D₂O): δ 1.74 (s, 3H), 3.32 (t, 2H), 4.08 (t, 2H), 5.54 (s, 1H), 5.95 (s, 1H); ¹³C-NMR (d₆-DMSO): δ 18.1, 38.3, 63.6, 126.0, 135.8, 157.4, 166.6, 182.7.

### Example 6. Synthesis of MOI-EG adduct of acethydrazide (Monomer F)

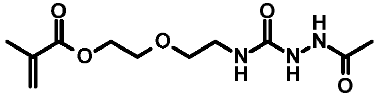

The general procedure described in Example 3 was followed with the exceptions that MOI-EG was used in place of IEM, and the monomer solution was not diluted after completion of the reaction. ¹H-NMR (D₂O): δ 1,76 (s, 3H), 1.86 (s, 3H), 3.18 (t, 2H), 3.47 (t, 2H), 3.64 (t, 2H), 4.15 (t, 2H), 5.56 (s, 1H), 5.98 (s, 1H). ¹³C-NMR (d₆-DMSO): δ 19.0, 21.3, 40.1, 65.0, 69.1, 70.4, 127.8, 136.6, 159.9, 168.6, 172.6.

### Example 7. Synthesis of IEM adduct of carbohydrazide (Monomer G)

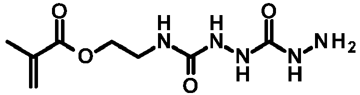

Carbohydrazide (1.125 grams, 0.0125 mol) was charged to a 250 mL round bottom flask, dissolved in 12.5 mL deionized water, and cooled in ice-water bath with magnetic stirring. IEM (1.75 mL, 0.0125 mol) was added by micropipette. Within four minutes, a colorless precipitate appeared. The reaction mixture was stirred for a total of thirty minutes. The ice-water bath was removed and the reaction was allowed to warm to room temperature while stirring for an additional hour. Chilled deionized water was then added to disperse the precipitated solid. The solid was filtered and dried provide 2.57 grams of Monomer G. ¹³C-NMR (d₆-DMSO): δ 18.0, 38.1, 63.7, 126.0, 135.8, 158.8, 160.2, 166.6.

### Example 8. Synthesis of IEM adduct of benzohydroxamic acid (Monomer H)

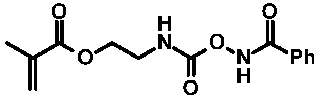

Benzohydroxamic acid (1.71 grams, 0.0125 mol) was charged to a 100 mL round bottom flask and dissolved in 50 mL ethyl acetate with magnetic stirring under a slow nitrogen stream. IEM (1.80 mL) was added by micropipette. The mixture was stirred for twenty minutes to provide a homogeneous yellow solution. A portion of the solution (20 mL) was withdrawn for analysis. The solvent was removed using a rotary evaporator and the residue was triturated with diethyl ether. The resulting off white solid was filtered and dried to provide 1.05 grams of Monomer H. ¹H-NMR (CDCl₃): δ 1.90 (s, 3H), 3.51 (m, 2H), 4.22 (t, 2H), 5.55 (s, 1H), 6.10 (s, 1H), 6.32 (br. t, 1H), 7.39 (m, 2H), 7.50 (m, 1H), 7.78 (d, 2H). ¹³C-NMR (CDCl₃): δ 13.1, 35.4, 58.0, 121.2, 122.2, 123.5, 125.2, 127.3, 130.5, 150.4, 162.1.

### Example 9. Synthesis of IEM adduct of methylcarbazate (Monomer J)

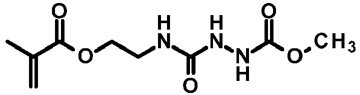

Methyl carbazate (methyl hydrazinocarboxylate, 1.126 grams, 0.0125 mol) was charged to a round bottom flask and dissolved in 12.5 mL of deionized water. The flask was placed in an ice-water bath. IEM (1.75 mL, 0.0125 mol) was added by micropipette with rapid magnetic stirring. After 10 minutes the bath was removed. Over the next 50 minutes, the insoluble droplets of IEM slowly disappeared and a colorless solid precipitated from the solution. The reaction mixture was stirred overnight. The colorless solid was recovered by filtration and dried to provide 2.0 grams of Monomer J. ¹H-NMR (D₂O): δ 1.74 (s, 3H) 3.31 (t, 2H), 3.55 (s, 3H), 4.05 (t, 2H), 5.54 (s, 1H), 5.95 (s, 1H). ¹³C-NMR (d₆-DMSO): δ 18.3, 38.4, 52.1, 64.0, 126.3, 136.1, 157.7, 158.8, 167.0.

### Example 10. Synthesis of VDM adduct of benzohydroxamic acid (Monomer I)

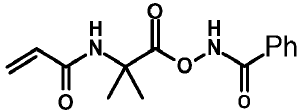

Monomer I can be made by the procedure described in Example 8, with the exception that IEM is replaced by VDM (1.57 mL).

### Example 11.

Coating solutions consisting of various molar ratios of the IEM adduct of 4-aminobutyric acid (IEM/GABA) and Monomer C were UV grafted to nylon membranes using the coating and UV grafting method described above. The functionalized membranes were analyzed for Static IgG binding capacity and the molar ratios of ligands/protein were calculated. Results are reported in Table 1 relative to those of the IEM/GABA homopolymer grafted membrane.

**Table 1.**

| Molar Ratio Charged (IEM/GABA:Monomer C) | Relative IgG Capacity | Relative Molar Ratio (calculated) GABA/protein |
|---|---|---|
| 100:0 | 1.00 | 1.00 |
| 90:10 | 0.94 | 0.85 |
| 80:20 | 1.03 | 0.92 |
| 70:30 | 0.86 | 0.87 |
| 50:50 | 0.70 | 0.85 |

### Example 12.

Example 11 was repeated, except that Monomer D was used in place of Monomer C. Results are reported in Table 2.

**Table 2.**

| Molar Ratio Charged (IEM/GABA:Monomer D) | Relative IgG Capacity | Relative Molar Ratio (calculated) GABA/protein |
|---|---|---|
| 100:0 | 1.00 | 1.00 |
| 90:10 | 0.92 | 0.95 |
| 80:20 | 0.92 | 0.90 |
| 70:30 | 0.86 | 0.86 |
| 50:50 | 0.71 | 0.89 |

### Comparative Example A.

Example 11 was repeated, except that methacrylic acid (MAA) was used in place of Monomer C. Results are reported in Table 3.

**Table 3.**

| Molar Ratio Charged (IEM/GABA: MAA) | Relative IgG Capacity | Relative Molar Ratio (calculated) | |
|---|---|---|---|
| | | Carboxyl group/protein | GABA/protein |
| 100:0 | 1.00 | 1.00 | 1.00 |
| 90:10 | 0.96 | 1.32 | 1.19 |
| 80:20 | 0.89 | 1.48 | 1.16 |
| 70:30 | 0.71 | 2.05 | 1.14 |
| 50:50 | 0.52 | 3.25 | 1.53 |

The results from Examples 11 and 12, as compared to Comparative Example A, indicate that protein binding capacity decreases less rapidly with increasing comonomer incorporation for monomers C and D than it does for MAA as a comonomer. This is quite surprising since MAA is a charged comonomer and the inventive monomers are neutral. In addition, the stoichiometry between ligand and protein decreases for the inventive monomers (i.e., the protein binding becomes for efficient) whereas it increases when MAA is the comonomer. In other words, when MAA is a comonomer, more ligands are required to bind the same amount of protein while the opposite is true with the inventive comonomers.

Various modifications and alterations to this disclosure will become apparent to those skilled in the art without departing from the scope of this disclosure. It should be understood that this disclosure is not intended to be unduly limited by the illustrative embodiments and examples set forth herein and that such examples and embodiments are presented by way of example only with the scope of the disclosure intended to be limited only by the claims set forth herein as follows.

## Claims

1. A monomer represented by the following general Formula (I):
CH₂=CR¹-C(=O)-X-R²-Z-X³-NR³-C(=X²)-X¹-R⁴ (I)
wherein:
R¹ is H or -CH₃;
R² is a (hetero)hydrocarbylene;
X is -O- or -NH-;
X¹ is -O-, -S-, -NH-, or a single bond;
X² is -O- or -S-;
X³ is -O- or -NR⁵-;
R⁴ is hydrogen, (hetero)hydrocarbyl, or -N(R³)₂;
each R³ and R⁵ is independently hydrogen or a (hetero)hydrocarbyl; and
Z is -C(=O)- or -NH-C(=O)-.

2. The monomer of claim 1 wherein R⁴ is methyl or phenyl.

3. The monomer of claim 1 or 2 wherein X² is -O-.

4. The monomer of any of claims 1 through 3 wherein X³ is -NR⁵-.

5. The monomer of any of claims 1 through 4 wherein Z is equal to -C(=O)-.

6. The monomer of any of claims 1 through 4 wherein Z is equal to -NH-C(=O)-.

7. The monomer of claim 5 or 6 wherein the group -X³-NR³-C(=X²)-X¹-R⁴ in Formula (I) is of formula
R⁴-NH-C(=X²)-N(R³)-N(R⁵)-,
R⁴-C(=X²)-N(R³)-N(R⁵)-,
R⁴-C(=X²)-N(R³)-,
R⁴-O-C(=O)-N(R³)-N(R⁵)-,
R⁴-S-C(=S)-N(R³)-N(R⁵)-, or
R⁴-NH-C(=X²)-N(R³)-.

8. A polymer comprising interpolymerized units of at least one monomer of any of the previous claims.

9. The polymer of claim 8 which is a copolymer.

10. The polymer of claim 9 which is a copolymer further comprising interpolymerized units of at least one monomer of the following general Formula (XI):
CH₂=CR¹¹-C(=O)-X-R¹²-[Z¹-R¹²]ₙ-L (XI)
wherein:
R¹¹ is selected from hydrogen, alkyl, aryl, and combinations thereof;
each R¹² is independently a (hetero)hydrocarbylene;
X is -O- or -NR¹³-, where R¹³ is hydrogen or a (hetero)hydrocarbyl;
Z¹ is a (hetero)hydrocarbylene comprising at least one hydrogen bond donor, at least one hydrogen bond acceptor, or a combination thereof;
n is an integer of 0 or 1; and
L is a heteroatom-containing group comprising at least one monovalent ligand functional group selected from acidic groups, basic groups, and salts thereof.

11. The polymer of claim 9 or 10 comprising 5 wt-% to 75 wt-% interpolymerized units of at least one monomer represented by Formula (I).

12. A copolymer comprising:
5 wt-% to 75 wt-% interpolymerized units of at least one monomer represented by the following general Formula (XII):
CH₂=CR²¹-C(=O)-X²⁰-R²²-Z²⁰-X²³-Y²⁰ (XII)
wherein:
R²¹ is H or -CH₃;
R²² is a (hetero)hydrocarbylene;
X²⁰ is -O- or -NH-;
X²³ is -O- or -NR²⁵-;
Z²⁰ is -C(=O)- or -NH-C(=O)-;
Y²⁰ is hydrogen, (hetero)hydrocarbyl, or -NR²³-C(=X²²)-X²¹-R²⁴;
X²¹ is -O-, -S-, -NH-, or a single bond;
X²² is -O- or -S-;
R²⁴ is hydrogen, (hetero)hydrocarbyl, or -N(R²³)₂; and
each R²³ and R²⁵ is independently hydrogen or a (hetero)hydrocarbyl; and
25 wt-% to 95 wt-% interpolymerized units of at least one monomer of the following general Formula (XI):
CH₂=CR¹¹-C(=O)-X-R¹²-[Z¹-R¹²]ₙ-L (XI)
wherein:
R¹¹ is selected from hydrogen, alkyl, aryl, and combinations thereof;
each R¹² is independently a (hetero)hydrocarbylene;
X is -O- or -NR¹³-, where R¹³ is hydrogen or a (hetero)hydrocarbyl;
Z¹ is a (hetero)hydrocarbylene comprising at least one hydrogen bond donor, at least one hydrogen bond acceptor, or a combination thereof;
n is an integer of 0 or 1; and
L is a heteroatom-containing group comprising at least one monovalent ligand functional group selected from acidic groups, basic groups, and
salts thereof.

13. The copolymer of claim 12 grafted to a substrate.

14. An article for biomaterial capture comprising
a porous polymeric substrate; and
borne on the porous substrate, a polymer of any one of claims 8 through 13.

15. The article of claim 14 wherein the polymer is grafted to the porous substrate.

## Patentansprüche

1. Ein Monomer, das durch die folgende allgemeine Formel (I) dargestellt ist:
CH₂=CR¹-C(=O)-X-R²-Z-X³-NR³-C(=X²)-X¹-R⁴ (I)
wobei:
R¹ H oder -CH₃ ist;
R² ein (Hetero)hydrocarbylen ist;
X -O- oder -NH- ist;
X¹ -O-, -S-, -NH- oder eine Einfachbindung ist;
X² -O- oder -S- ist;
X³ -O- oder -NR⁵- ist;
R⁴ Wasserstoff, (Hetero)hydrocarbyl oder -N(R³)₂ ist;
jedes R³ und R⁵ unabhängig Wasserstoff oder ein (Hetero)hydrocarbyl ist; und
Z -C(=O)- oder -NH-C(=O)- ist.

2. Das Monomer nach Anspruch 1, wobei R⁴ Methyl oder Phenyl ist.

3. Das Monomer nach Anspruch 1 oder 2, wobei X² -O- ist.

4. Das Monomer nach einem der Ansprüche 1 bis einschließlich 3, wobei X³ -NR⁵- ist.

5. Das Monomer nach einem der Ansprüche 1 bis einschließlich 4, wobei Z gleich -C(=O)- ist.

6. Das Monomer nach einem der Ansprüche 1 bis einschließlich 4, wobei Z gleich -NH-C(=O)- ist.

7. Das Monomer nach Anspruch 5 oder 6, wobei die Gruppe -X³-NR³-C(=X²)-X¹-R⁴ in Formel (I) ist von Formel
R⁴-NH-C(=X²)-N(R³)-N(R⁵)-,
R⁴-C(=X²)-N(R³)-N(R⁵)-,
R⁴-C(=X²)-N(R³)-,
R⁴-O-C(=O)-N(R³)-N(R⁵)-,
R⁴-S-C(=S)-N(R³)-N(R⁵)- oder
R⁴-NH-C(=X²)-N(R³)-.

8. Ein Polymer, umfassend interpolymerisierte Einheiten von mindestens einem Monomer nach einem der vorstehenden Ansprüche.

9. Das Polymer nach Anspruch 8, das ein Copolymer ist.

10. Das Polymer nach Anspruch 9, das ein Copolymer ist, ferner umfassend interpolymerisierte Einheiten von mindestens einem Monomer der folgenden allgemeinen Formel (XI):
CH₂=CR¹¹-C(=O)-X-R¹²-[Z¹-R¹²]ₙ-L (XI)
wobei:
R¹¹ aus Wasserstoff, Alkyl, Aryl und Kombinationen davon ausgewählt ist;
jedes R¹² unabhängig ein (Hetero)hydrocarbylen ist;
X -O- oder -NR¹³- ist, wobei R¹³ Wasserstoff oder ein (Hetero)hydrocarbyl ist;
Z¹ ein (Hetero)hydrocarbylen ist, umfassend mindestens einen Wasserstoffbindungsdonor, mindestens einen Wasserstoffbindungsakzeptor oder eine Kombination davon;
n eine ganze Zahl von 0 oder 1 ist; und
L eine heteroatomhaltige Gruppe ist, umfassend mindestens eine einwertige Liganden-funktionelle Gruppe, die aus sauren Gruppen, basischen Gruppen und Salzen davon ausgewählt ist.

11. Das Polymer nach Anspruch 9 oder 10, umfassend zu 5 Gew.-% bis 75 Gew.-% interpolymerisierte Einheiten von mindestens einem Monomer, das durch Formel (I) dargestellt ist.

12. Ein Copolymer, umfassend:
zu 5 Gew.-% bis 75 Gew.-% interpolymerisierte Einheiten von mindestens einem Monomer, das durch die folgende allgemeine Formel (XII) dargestellt wird:
CH₂=CR²¹-C(=O)-X²⁰-R²²-Z²⁰-X²³-Y²⁰ (XII)
wobei:
R²¹ H oder -CH₃ ist;
R²² ein (Hetero)hydrocarbylen ist;
X²⁰ -O- oder -NH- ist;
X²³ -O- oder -NR²⁵- ist;
Z²⁰ -C(=O)- oder -NH-C(=O)- ist;
Y²⁰ Wasserstoff, (Hetero)hydrocarbyl oder -NR²³-C(=X²²)-X²¹-R²⁴ ist;
X²¹ -O-, -S-, -NH- oder eine Einfachbindung ist;
X²² -O- oder -S- ist;
R²⁴ Wasserstoff, (Hetero)hydrocarbyl oder -N(R²³)₂ ist; und
jedes R²³ und R²⁵ unabhängig Wasserstoff oder ein (Hetero)hydrocarbyl ist; und
zu 25 Gew.-% bis 95 Gew.-% interpolymerisierte Einheiten von mindestens einem Monomer der folgenden allgemeinen Formel (XI):
CH₂=CR¹¹-C(=O)-X-R¹²-[Z¹-R¹²]ₙ-L (XI)
wobei:
R¹¹ aus Wasserstoff, Alkyl, Aryl und Kombinationen davon ausgewählt ist;
jedes R¹² unabhängig ein (Hetero)hydrocarbylen ist;
X -O- oder -NR¹³- ist, wobei R¹³ Wasserstoff oder ein (Hetero)hydrocarbyl ist;
Z¹ ein (Hetero)hydrocarbylen ist, umfassend mindestens einen Wasserstoffbindungsdonor, mindestens einen Wasserstoffbindungsakzeptor oder eine Kombination davon;
n eine ganze Zahl von 0 oder 1 ist; und
L eine heteroatomhaltige Gruppe ist, umfassend mindestens eine einwertige Liganden-funktionelle Gruppe, die aus sauren Gruppen, basischen Gruppen und Salzen davon ausgewählt ist.

13. Das Copolymer nach Anspruch 12, das auf ein Substrat gepfropft ist.

14. Ein Artikel für eine Biomaterialerfassung, umfassend
ein poröses Polymersubstrat; und
getragen auf dem porösen Substrat, ein Polymer nach einem der Ansprüche 8 bis einschließlich 13.

15. Der Artikel nach Anspruch 14, wobei das Polymer auf das poröse Substrat gepfropft wird.

## Revendications

1. Monomère représenté par la Formule générale (I) suivante:
CH₂=CR¹-C(=O)-X-R²-Z-X³-NR³-C(=X²)-X¹-R⁴ (I)
dans lequel:
R¹ est H ou -CH₃;
R² est un (hétéro)hydrocarbylène;
X est -O- ou -NH-;
X¹ est -O-, -S-, -NH-, ou une liaison simple;
X² est -O- ou -S-;
X³ est -O- ou -NR⁵-;
R⁴ est hydrogène, (hétéro)hydrocarbyle, ou -N(R³)₂;
chaque R³ et R⁵ est indépendamment hydrogène ou un (hétéro)hydrocarbyle; et
Z est -C(=O)-ou -NH-C(=O)-.

2. Monomère selon la revendication 1, dans lequel R⁴ est méthyle ou phényle.

3. Monomère selon la revendication 1 ou 2, dans lequel X² est -O-.

4. Monomère selon l'une quelconque des revendications 1 à 3, dans lequel X³ est -NR⁵-.

5. Monomère selon l'une quelconque des revendications 1 à 4, dans lequel Z est égal à -C(=O)-.

6. Monomère selon l'une quelconque des revendications 1 à 4, dans lequel Z est égal à -NH-C(=O)-.

7. Monomère selon la revendication 5 ou 6, dans lequel le groupe -X³-NR³-C(=X²)-X¹-R⁴ dans la Formule (I) est de formule
R⁴-NH-C(=X²)-N(R³)-N(R⁵)-,
R⁴-C(=X²)-N(R³)-N(R⁵)-,
R⁴-C(=X²)-N(R³)-,
R⁴-O-C(=O)-N(R³)-N(R⁵)-,
R⁴-S-C(=S)-N(R³)-N(R⁵)-, ou
R⁴-NH-C(=X²)-N(R³)-.

8. Polymère comprenant des motifs interpolymérisés d'au moins un monomère selon l'une quelconque des revendications précédentes.

9. Polymère selon la revendication 8 qui est un copolymère.

10. Polymère selon la revendication 9 qui est un copolymère comprenant en outre des motifs interpolymérisés d'au moins un monomère de la Formule générale (XI) suivante:
CH₂=CR¹¹-C(=O)-X-R¹²-[Z¹-R¹²]ₙ-L (XI)
dans lequel:
R¹¹ est choisi parmi hydrogène, alkyle, aryle, et des combinaisons de ceux-ci;
chaque R¹² est indépendamment un (hétéro)hydrocarbylène;
X est -O- ou -NR¹³-, où R¹³ est hydrogène ou un (hétéro)hydrocarbyle;
Z¹ est un (hétéro)hydrocarbylène comprenant au moins un donneur de liaison hydrogène, au moins un accepteur de liaison hydrogène, ou une combinaison de ceux-ci;
n est un nombre entier de 0 ou 1; et
L est un groupe contenant un hétéroatome comprenant au moins un groupe fonctionnel ligand monovalent choisi parmi des groupes acides, des groupes basiques, et des sels de ceux-ci.

11. Polymère selon la revendication 9 ou 10, comprenant 5% en poids à 75% en poids de motifs interpolymérisés d'au moins un monomère représenté par la Formule (I).

12. Copolymère comprenant:
5% en poids à 75% en poids de motifs interpolymérisés d'au moins un monomère représenté par la Formule générale (XII) suivante:
CH₂=CR²¹-C(=O)-X²⁰-R²²-Z²⁰-X²³-Y²⁰ (XII)
dans lequel:
R²¹ est H ou -CH₃;
R²² est un (hétéro)hydrocarbylène;
X²⁰ est -O- ou -NH-;
X²³ est -O- ou -NR²⁵-;
Z²⁰ est -C(=O)- ou -NH-C(=O)-;
Y²⁰ est hydrogène, (hétéro)hydrocarbyle, ou -NR²³-(=X²²)-X²¹-R²⁴;
X²¹ est -O-, -S-, -NH-, ou une liaison simple;
X²² est -O- ou -S-;
R²⁴ est hydrogène, (hétéro)hydrocarbyle, ou -N(R²³)₂; et
chaque R²³ et R²⁵ est indépendamment hydrogène ou un (hétéro)hydrocarbyle; et
25% en poids à 95% en poids de motifs interpolymérisés d'au moins un monomère de la Formule générale (XI) suivante:
CH₂=CR¹¹-C(=O)-X-R¹²-[Z¹-R¹²]ₙ-L (XI)
dans lequel:
R¹¹ est choisi parmi hydrogène, alkyle, aryle, et des combinaisons de ceux-ci;
chaque R¹² est indépendamment un (hétéro)hydrocarbylène;
X est -O- ou -NR¹³-, où R¹³ est hydrogène ou un (hétéro)hydrocarbyle;
Z¹ est un (hétéro)hydrocarbylène comprenant au moins un donneur de liaison hydrogène, au moins un accepteur de liaison hydrogène, ou une combinaison de ceux-ci;
n est un nombre entier de 0 ou 1; et
L est un groupe contenant un hétéroatome comprenant au moins un groupe fonctionnel ligand monovalent choisi parmi des groupes acides, des groupes basiques, et des sels de ceux-ci.

13. Copolymère selon la revendication 12 greffé sur un substrat.

14. Article pour capture de biomatériau comprenant
un substrat polymère poreux; et
porté sur le substrat poreux, un polymère selon l'une quelconque des revendications 8 à 13.

15. Article selon la revendication 14, dans lequel le polymère est greffé sur le substrat poreux.
